(19) Europäisches Patentamt
European Patent Office
Office européen des brevets

(11) **EP 3 872 711 A1**

(12) **EUROPEAN PATENT APPLICATION**

(43) Date of publication:
**01.09.2021 Bulletin 2021/35**

(51) Int Cl.:
***G06N 3/04*** (2006.01)          ***G06N 3/08*** (2006.01)
***G06N 20/00*** (2019.01)

(21) Application number: **20160197.8**

(22) Date of filing: **28.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sensyne Health Group Limited Oxford OX4 4GE (GB)**

(72) Inventors:
- **IRVING, Benjamin John**
  **Oxford, OX4 4GE (GB)**
- **CARR, Oliver Sebastian Leslie**
  **Oxford, OX4 4GE (GB)**
- **JOVANOVIC, Stojan**
  **Oxford, OX4 4GE (GB)**

(74) Representative: **Korenberg, Alexander Tal et al Kilburn & Strode LLP Lacon London 84 Theobalds Road London WC1X 8NL (GB)**

(54) **SEMI-SUPERVISED MACHINE LEARNING METHOD AND SYSTEM SUITABLE FOR IDENTIFICATION OF PATIENT SUBGROUPS IN ELECTRONIC HEALTHCARE RECORDS**

(57)    A computer-implemented method of training an artificial neural network is provided. The artificial neural network comprises a feedforward autoencoder and a classification layer, the feedforward autoencoder comprising an input layer of neurons, an output layer of neurons, an embedding layer of neurons between the input and output layers, one or more non-linear intermediate layers of neurons between the input and embedding layer, one or more further non-linear intermediate layers of neurons between the embedding and output layer and respective network weights for each layer, wherein the number of neurons in the embedding layer is less than the number of neurons in the input layer. Data units are defined within a data space to have a data value for each dimension of the data space and a classification label associated with each data unit indicating one of a plurality of groups to which each data sample belongs. The network is trained using a combination of unsupervised learning to reduce a reconstruction loss of the autoencoder and supervised learning to reduce a classification loss of the classification layer. The resulting network finds application in providing embeddings for classification or clustering, for example in the context of analysing phenotypical data, such as physiological data, which may be obtained from electronic health records. The classification or clustering may be used to predict or identify pathologies or to analyse patient subgroups to analyse, for example treatment efficacy, outcomes or survival rates or to triage patients in accordance with relevant criteria.

Fig. 3B

## Description

### TECHNICAL FIELD

The present disclosure relates to a machine learning system and method for embedding data using a combination of unsupervised and supervised learning, and in particular for clustering data using the embedding, for example clustering patient subgroups based on electronic healthcare records (EHR).

### BACKGROUND

Identifying different patient subgroups is a fundamental step in understanding disease phenotypes and is necessary for improving treatment and care of patients. One way this can be achieved is by clustering patients into subgroups using EHR records including vital signs, blood tests, treatment and/or disease classification. EHR data is heterogenous, high-dimensional and sparse and therefore does not lend itself well to traditional clustering approaches such as k-means, mean shift or gaussian mixture models. It would be advantageous to provide systems and methods which address one or more of these problems, in isolation or in combination.

One way to improve the accuracy and efficiency of clustering high-dimensional data such as EHR is by finding a low-dimensional embedding and performing the clustering task on the embedded data. Finding a lower dimensional embedding is commonly achieved by linear approaches such as principal component analysis. Alternatively, machine learning models such as autoencoders have been shown to allow a more complex representation of data. Deep embedded clustering (DEC) is a technique that combines autoencoders with a clustering loss (or error) to find a low-dimensional embedding that is both representative of the data and provides optimal clusters. *See* Xie, Junyuan, Ross Girshick, and Ali Farhadi. "Unsupervised deep embedding for clustering analysis." International conference on machine learning. 2016, incorporated by reference in this disclosure. Nevertheless, while DEC learns a low-dimensional separable representation, the key challenge of facilitating the discovery of unknown subgroups remains unaddressed. Improved methods of learning an embedding are needed, in the context of analysing EHR but also more widely in any other application domain where it is of interest to cluster high-dimensional data.

### SUMMARY

In a first aspect, a computer-implemented method of training an artificial neural network is provided. The artificial neural network comprises a feedforward autoencoder and a classification layer, the feedforward autoencoder comprising an input layer of neurons, an output layer of neurons, an embedding layer of neurons between the input and output layers, one or more non-linear intermediate layers of neurons between the input and embedding layer, one or more further non-linear intermediate layers of neurons between the embedding and output layer and respective network weights for each layer, wherein the number of neurons in the embedding layer is less than the number of neurons in the input layer. Data units are defined within a data space to have a data value for each dimension of the data space and a classification label associated with each data unit indicating one of a plurality of groups to which each data sample belongs.

The method comprises applying the data values of each data unit to corresponding neurons of the input layer to produce an output of the neurons in the output layer. Neuron outputs of the neurons in the embedding layer resulting from the application of each data unit provide an embedding of the data unit. The embedding has fewer dimensions than the data space. The method further comprises using the embedding of each data unit to provide, directly or via one or more intermediate layers, an input to the classification layer to produce a classification for each data unit as an output of the classification layer, comparing the classification and classification label to determine a classification loss for each data unit and comparing the data values and the output of the respective neurons in the output layer to determine a reconstruction loss for each data unit. The comparisons are used to train the artificial neural network to reduce the reconstruction loss and the classification loss across the data units.

Advantageously, by combining the unsupervised training of the autoencoder with the supervised training of the classification layer, it has been found that the weights of the autoencoder are adapted to provide an embedding that better separates the class structure in the data and is therefore more useful for use in classification tasks. Surprisingly, it has further been found that this can further improve the usefulness of the embedding for clustering tasks that can uncover previously unidentified (unlabelled) groupings in the data.

The autoencoder may be trained to use the reconstruction loss in any suitable way. In some embodiments, this may include corrupting the input, for example by adding noise, such as normal distributed independent noise, to each data value or by dropping data values, setting them to zero, for example at random (denoising autoencoder). The training may be done in a single phase or the autoencoder may be trained as a stacked autoencoder by greedily training each hidden layer to be an embedding layer of an autoencoder comprising the previous layer as an input layer until all layers in the stacked encoder have been trained greedily in this way, followed by adding the decoder as the reverse order of layers of the stacked encoder and fine-tuning the reconstruction loss by back-propagating it through the resulting full autoencoder architecture to adjust the weights of the full structure in a refining phase. Denoising may be applied during the greedy stacking phase, during the refining phase or both, or the autoencoder may be trained with greedy stacking without denoising. In denoising, the reconstruction loss is typically calculated using the un-corrupted input without noise added / values dropped.

The method may comprise using the respective embeddings of the data units as an input to a clustering algorithm to assign each data unit to a respective one of a plurality of clusters by optimising a clustering cost function. Training the artificial neural network may further comprise using a value of the clustering cost function for each data unit to train the autoencoder. The clustering cost function may comprise a target class assignment probability distribution, emphasising high-confidence class assignments and penalising large clusters.

The training may proceed in a number of ways. For example, where applicable, training using the clustering cost function may be done in a separate phase subsequent to training on the reconstruction and classification losses. Similarly, whether a clustering cost is used or not, training on the classification loss may occur in a separate phase subsequent to training on the reconstruction loss. Training may comprise updating all layers of the autoencoder to reduce the reconstruction loss and fixing at least one of the intermediate layers while updating the remaining layers of the artificial neural network to reduce the classification loss subsequent to training the autoencoder to reduce the reconstruction loss. It will be appreciated that in any of the disclosed aspects or embodiments, once training of the autoencoder has been completed, the decoder part of the autoencoder can be discarded, for example during subsequent training using a classification loss or clustering cost, or during use of the artificial network to compute an embedding for classification or clustering subsequent to training.

Alternatively or additionally, training the artificial neural network comprises alternating between training epochs reducing the reconstruction loss and training epochs reducing the classification loss or, where applicable, interleaving respective training epochs reducing the reconstruction loss, reducing the classification loss and using the clustering cost function. Similarly, training the artificial neural network may comprise using a combined cost function combining the reconstruction loss and the classification loss to train the artificial neural network or, where applicable, training the artificial neural using a combined cost function combining the reconstruction loss, the classification loss and the clustering cost function.

The data units may be representative of phenotypic data of respective patients from a population of patients, wherein the population comprises at least two groups, for example mutually exclusive groups, and the data label identifies the patient belongs to. The at least two groups may comprise a pathology group of patients with an identified pathology, for example heart failure, and a control group of patients without the pathology. The phenotypic data is obtained from an electronic health record and/or may be physiological data. The physiological data may comprise three or more of: systolic blood pressure, diastolic blood pressure, heart rate, oxygen saturation, temperature, alanine aminotransferase, creatinine, c-reactive protein, platelets, potassium, sodium, urea and white blood cells.

Further aspects comprise a computer-implemented method of classifying a data unit as belonging to a group of data units of a plurality of groups of data units. The method comprises providing a neural network trained as described above, applying the data unit to the input layer, obtaining the embedding for the data unit and using the embedding to classify the data unit. In this aspect, the embedding used during training of the artificial neural network is used for classifying a new data unit, for example from a new patient, for class belonging, for example the presence of a pathology. The new data unit may be previously unseen or at least not used a part of the training.

Yet further aspects comprise a computer implemented method of identifying subgroups comprising training a neural network in accordance with the methods described above. The method comprises clustering the embeddings of the data units into a plurality of clusters and identifying a subgroup, for example a specific presentation of a pathology, of at least one of the groups, for example patients with a specific pathology or outcome, as corresponding to one of the clusters. The method may comprise clustering the embeddings of the data units into a plurality of clusters and determining a survival rate for each cluster. The clustering may be done using the clustering algorithm used for training, where applicable, or using a different clustering algorithm, for example agglomerative hierarchical clustering, with the embeddings as input. The method may comprise classifying a new data unit, for example a new data unit as described above, as belonging to one of the subgroups.

Advantageously, identifying subgroups in this way enables a better estimation of responsiveness to treatment, survival rates or other quantities of interest, based on belonging to a particular subgroup. For example, survival rates or treatment effectiveness can be measured in a group and analysed by subgroup. Identified subgroup may be used as part of a drug development pipeline to separately assess responses to a drug trial in respective subgroups, which may help to more specifically target development or identify more specific uses of a new drug then when looking at a, for example pathology, population group as a whole. In another example, related conditions identified in subgroups can be used as exclusion criteria for treatment or trials for patients that might have poorer outcomes under treatment. In other examples, identifying subgroups can be used to triage patients as to criticality or urgency of a condition or to propose a treatment plan.

Aspects extend to one or more computer-readable media encoding computer instruction that, when executed on a computing device, implement methods as described above and to a system comprising the one or more computer readable media, a memory for storing the artificial neural network and the data units and a processor for executing the instructions.

The present disclosure refers to artificial neural networks. It will be appreciated that artificial neural networks represent a specific parametrization of a non-linear function (in terms of network weights) and that the present disclosure is not limited by the language used to describe the non-linear function or its structure. It will be understood that an artificial neural network in the context of a computer implemented invention refers to a physical entity that exists either in terms of a physical state of a general purpose or specifically adapted computing platform or in a specifically adapted physical circuitry, for example.

## BRIEF DESCRIPTION OF THE FIGURES

Illustrative implementations of the present disclosure will now be described, by way of example only, with reference to the drawings. In the drawings:

> **Figure 1** schematically depicts a process of clustering data in an embedded space in accordance with aspects of the present specification;
> **Figure 2** shows a schematic diagram of an autoencoder network in accordance with aspects of the present specification;
> **Figure 3A** shows a process of processing data to learn an embedding according to some embodiments;
> **Figure 3B** shows a schematic diagram of an autoencoder network in accordance with aspects of the present specification;
> **Figure 3C** shows a process of processing data to learn an embedding according to some embodiments;
> **Figure 4** shows a schematic visualisation of an embedding learnt according to an embodiment and of an embedding learnt using DEC;
> **Figure 5** shows scatterplots of embeddings of an example EHR dataset representing heart failure and control groups using DEC (5A) and semi-supervised clustering according to an embodiment (5B);
> **Figure 6** shows the Area Under the Receiver Operating Curve (AUROC) results of discriminating between the heart failure and control groups using three low-dimensionality embeddings: a semi-supervised embedding according to an embodiment, a DEC unsupervised embedding with a random forest classifier and a PCA embedding with a random forest classifier;
> **Figure 7** shows the unknown patient subgroups discovered using a semi-supervised clustering approach according to an embodiment;
> **Figure 8** shows survival curves for the patient subgroups in Figure 7; and
> **Figure 9** illustrates a block diagram of one implementation of a computing device.

## DETAILED DESCRIPTION

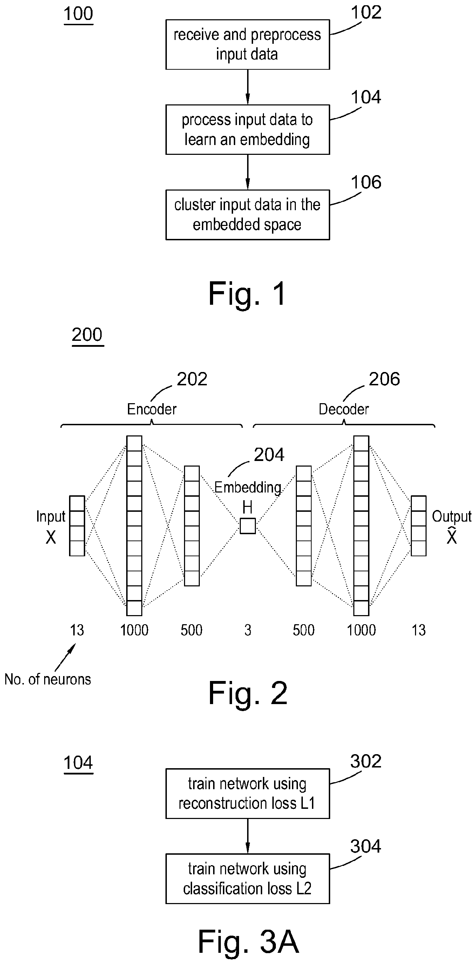

Figure 1 schematically depicts a process 100 of clustering data in an embedded space to discover unknown subgroups in the data. The data may relate to any type of data that can be classified into subgroups. For example, the data may refer to physiological data from a population of patients that can be divided into patient subgroups. Physiological data can include but not be limited to vital signs or laboratory measurements. Process 100 can be implemented by any kind of general computer and is specifically suited for computers with a processing capacity adapted for machine learning processes.

The process comprises a step 102 of receiving an input data structure X that stores the data to be clustered. The input data structure X comprises instances i and input values $x_i$ for each instance $i$. By way of non-limiting example, the input data structure may comprise electronic health records (EHR) of one or more patient cohorts. EHR may comprise physiological measurements such as vital signs or laboratory measurements that include but are not limited to blood pressure, heart rate, oxygen saturation, temperature, alanine aminotransferase, creatinine, c-reactive protein, platelets, potassium, sodium, urea and white blood cells. In addition, EHR data may further comprise medical codes, medication information, procedural codes or text. Each instance $i$ of the input data structure X represents a patient and each input value $x_i$ represents one entry in the EHR of that patient.

Once received, the input data structure X may be preprocessed before being passed on to the next step of process 100. Preprocessing may include but is not limited to filling in missing input values or partial deletion of data. In the example of EHR data, the input values are often sparse as the vital signs and laboratory measurements required for an effective diagnosis vary greatly across different diseases. In this example, preprocessing may be performed to fill in missing values to ensure all patients have the same number of input values $x_i$ in the EHR data for clustering. Alternatively, this may be an intermediate representation that accounts for time between events (for example blood tests, diagnoses, medication and procedures) within or between admissions. At the end of step 102, the input data structure X is of dimensions $n$ and $m$, $n$ representing the total number of patients selected for analysis and $m$ representing the total number of EHR data points for each patient.

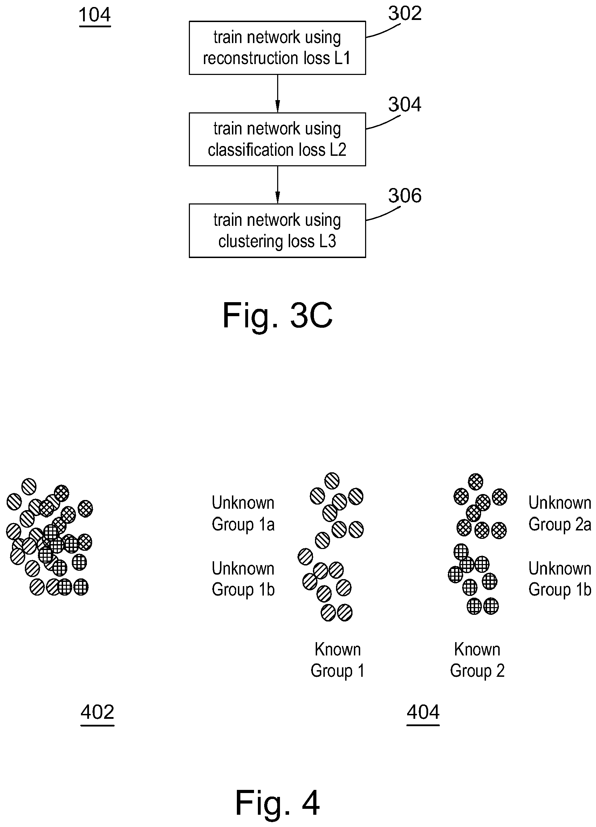

In step 104, the input data structure X is processed to learn a low-dimensional cluster-optimised embedding H that facilitates the discovery of unknown subgroups within the data using a semi-supervised machine learning model. In particular, the semi-supervised machine learning model refers to an autoencoder network 200, as illustrated in Figure 2. The autoencoder network 200 improves on existing embedding methods such as DEC by combining in a novel way unsupervised and supervised training of an autoencoder network using labels of known groups in the data. This approach facilitates the embedding being well-suited for clustering and may facilitate the discovery of unknown subgroups within the data, thus enabling an improved analysis of high-dimensional data. In some embodiments, step 104 is a two-stage process as described below with reference to Figure 3A. In other embodiments, step 104 is a three-stage process, as described below with reference to Figure 3C.

By way of non-limiting example, with reference to the EHR dataset, the known groups may refer to different diseases. Guided by this classification, the semi-supervised machine learning model learns an embedding that is tuned to the problem of separating known groups in the data. Surprisingly, inventors have found that this facilitates finding unknown patient subgroups within the known populations, for example within a cohort associated with a particular disease. Consequently, the present process can improve patient stratification and in this way facilitate the discovery of unknown subgroups or phenotypes of a particular disease through the cluster optimised embedding H.

In step 106, the input data is clustered in the embedded space into a plurality of clusters representing unknown subgroups. Performing clustering directly on high-dimensional data is challenging due to the problem known as "the curse of dimensionality", which refers to a series of phenomena that arise in high-dimensional spaces but do not occur in lower dimensions. By performing clustering on the low-dimensional embedded space, step 106 avoids the "curse of dimensionality" problem and improves the efficiency and accuracy of the clustering process. Step 106 may be implemented using any clustering approach, depending on the problem of interest. A clustering approach refers to any approach that groups a multi-dimensional data set into closely related groups and may refer to techniques such as k-nearest neighbours, k-means, Gaussian mixture models, mean shift and the like.

By way of non-limiting example, with reference to the EHR dataset, one category of clustering approaches well-suited to the optimised patient embedding H from step 104 may be hierarchical clustering approaches as they improve the understanding of the relationship between different patient subgroups. In this example, step 106 groups patients suffering from a particular disease into previously unknown subgroups for example based on common symptoms, leading to improved patient stratification and to the discovery of disease subtypes which may further lead to the improved treatment and care of patients in that group.

Autoencoder networks are a type of machine learning models commonly used for learning low-dimensional embeddings. Figure 2 illustrates an example of an autoencoder network 200. An autoencoder network is a specific type of an artificial neural network and comprises an encoder 202 and a decoder 206. The most common form of an autoencoder network is a feedforward multilayer perceptron with an input layer, one or more hidden layers and an output layer. Each layer is formed of one or more elementary units known as neurons and which implement a non-linear mathematical operation. A neuron receives inputs from one or more other neurons in the previous layer of the feedforward neural network, processes the inputs and then passes them on to other neurons in the next layer of the feedforward neural network. The neuron processes the inputs by computing a weighted sum of the inputs and then applying the non-linear mathematical operation, called an activation function, to the weighted sum before passing the result to the neurons in the next layer. The weights that the neuron applies to each received input represent parameters of the feedforward neural network. The parameters of the network are updated during training so that they best represent the data with respect to which the feedforward neural network is trained.

An autoencoder network is trained with the purpose of reconstructing its inputs X, and therefore the output layer of an autoencoder has the same number of neurons as the input layer. The encoder 202 is configured to encode the information stored in the input data X into a low-dimensional embedding H 204 representing the output of an embedding layer. The decoder 206 takes as an input the low-dimensional embedding H 204 and decodes the information in the embedding to provide a reconstruction $\hat{X}$ of the input data X. In the example autoencoder 200, the input and output layers have 13 neurons each, indicating that the dimensionality of the input data is 13. The hidden layers have 1000, 500, 3, 500 and 1000 neurons. The embedding layer has 3 neurons, meaning that the autoencoder networks learns to embed the 13 dimensions of the input data into 3 dimensions. In order to output a low-dimensional embedding, the embedding layer will generally have fewer neurons than the input layer and it will be appreciated that the disclosure is not limited to any particular number of neurons. Similarly, the disclosure is not limited to a particular number of layers and networks with a single intermediate hidden layer, or none, between the input and the embedding layer are equally envisaged. It will further be appreciated that the disclosure is not limited to the described autoencoder networks but other network architectures and learning methods. For example, the addition of noise to the autoencoder input may be omitted so that the autoencoder is trained as a conventional rather than a denoising autoencoder. Also, a variational autoencorder or other approaches may be used, for example a variational autoencoder. See Kingma, Diederik P., and Max Welling. "An introduction to variational autoencoders. " Foundations and Trends® in Machine Learning 12.4 (2019): 307-392, incorporated by reference in this disclosure. See also Vincent, Pascal, et al. "Stacked denoising autoencoders: Learning useful representations in a deep network with a local denoising criterion." Journal of machine learning research 11.Dec (2010): 3371-3408], incorporated by reference in this disclosure. Many training strategies can be used, for example pre-training pairs of layers of the encoder as Restricted Boltzman Machines, and then adding a decoder as the reverse of the encoder to train the resulting autoencoder on a reconstruction loss, for example using back-propagation or any suitable parameter optimizer. See Hinton, Geoffrey E., and Ruslan R. Salakhutdinov. "Reducing the dimensionality of data with neural networks." science 313.5786 (2006): 504-507, incorporated by reference in this disclosure.

Autoencoder network 200 is trained by minimising a reconstruction loss that measures the difference between the inputs X and the outputs $\hat{X}$ through updating the network weights of the autoencoder network 200. By aiming to minimise the difference between X and $\hat{X}$, the autoencoder network 200 maximises the amount of information captured in the embedding H 204 used to reconstruct the input data X. Autoencoders are unsupervised models as they do not require input labels during training and use the unlabelled inputs directly.

Autoencoder network 200 is trained using backpropagation, the most widely used algorithm in the fields of feedforward neural networks. The backpropagation algorithm comprises a forward and a backward pass through all the layers of the autoencoder network 200. In the forward pass, training instances are passed forward through all layers starting from the input layer, then through the hidden layers and finally to the output layer which produces an output for each training instance. At the end of the forward pass, a loss (or error) function L computes an error corresponding to each output. The loss function L of autoencoder network 200 is represented by the reconstruction loss. In the backward pass, the error is propagated backwards from the output layer to the input layer. During the backward pass, the weights of each neuron are updated in proportion to the neuron's contribution to the overall error calculated by computing the gradient of the loss function L with respect to that neuron. Therefore, during training, a network layer is updated once all the weights connecting neurons in that layer to neurons in the previous layer are updated. The parameter update is usually computed via an optimisation algorithm that minimises a function that in the case of neural networks is represented by the loss function L. Usually iterative optimisation algorithms such as gradient descent or modified versions of gradient descent such as the Adam optimiser are used (see Kingma, Diederik P., and Jimmy Ba. "Adam: A method for stochastic optimization."arXiv preprint arXiv:1412.6980 (2014), incorporated by reference in this disclosure). Minimising the loss function L of autoencoder network 200 is equivalent to driving the network outputs $\hat{X}$ to be as close as possible to the desired outputs, which in this case are represented by the corresponding inputs X. Once one forward pass and one backward pass is executed for all training instances, an epoch of training is completed. At the end of all epochs of training, the autoencoder network 200 will have learnt how to best represent the training data X through the embedding H 204. The number of epochs may be fixed or determined by a stopping criterion. In some embodiments, the autoencoder network 200 is implemented using a rectified linear unit function, otherwise known as ReLU, as the activation function for some of the layers.

The autoencoder network 200 is implemented as a denoising autoencoder by corrupting the input data X before reconstruction. In some embodiments, the input is corrupted by adding noise, for example normally distributed independent noise, to each instance of the input data structure. In some embodiments, the input is corrupted by dropping input data values by setting their respective weights to zero at random.

In some embodiments, the autoencoder network 200 is trained as a stacked autoencoder by greedily training each hidden layer to be an embedded layer of an autoencoder comprising the previous layer as an input layer until all layers in the stacked autoencoder have been trained greedily in this way. See Xie, Junyuan, Ross Girshick, and Ali Farhadi. "Unsupervised deep embedding for clustering analysis." International conference on machine learning. 2016, incorporated by reference in this disclosure. After all layers have been trained using the greedy layer-wise training, the autoencoder network 200 is fine-tuned by minimising the reconstruction loss using the backpropagation algorithm through the resulting full autoencoder network 200.

In some embodiments, denoising may be applied during the greedy stacking phase, during the refining phase or both, or the autoencoder may be trained with greedy stacking without denoising.

In some embodiments, step 104 is a two-stage process, as shown in Figure 3A. In step 302, the autoencoder network 200 is trained using an appropriate reconstruction loss L1 to learn the embedding H 204 of the input data. The reconstruction loss L1 may in some embodiments be defined as a mean-squared error training loss, in accordance with equation (1):

$$L1 = \frac{1}{n}\Sigma_i\left(X_i - \widehat{X_i}\right)^2 \qquad (1)$$

where $i \in \{1, 2 \dots n\}$ represents an instance of the input data X and the rest of the symbols are consistent with those defined above. As no labels are used, the training at step 302 is unsupervised.

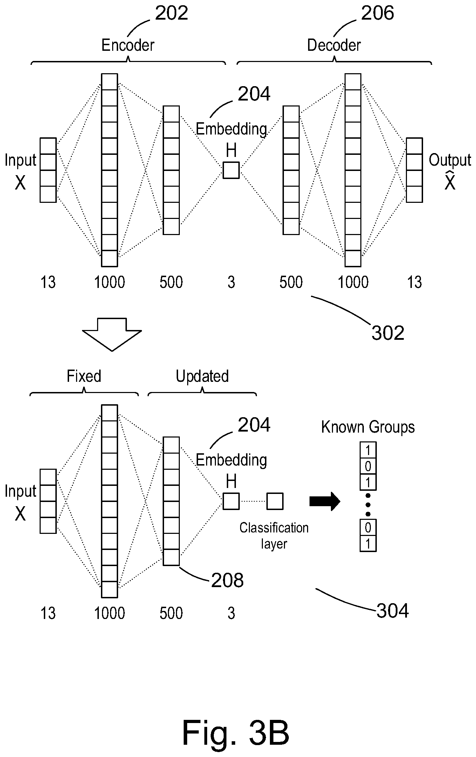

In step 304, once the unsupervised training using reconstruction loss L1 is finished, the encoder 202 and the embedding layer of the autoencoder are used with an additional classification layer, for example a fully-connected classification layer, to train a supervised classifier against known groups in the input data X using an appropriate classification loss L2. The additional classification layer outputs a predicted label $\hat{y}_i$, for each instance $i$ of the input data X and together with the encoder 202 forms the supervised classifier. During this step, only the embedding layer and the previous layer 208 of the encoder are updated, while the rest of the layers are kept fixed, as illustrated schematically in Figure 3B. It will be appreciated that in some instances some or all of the other layers may be allowed to vary, as well. The classification loss L2 may in some embodiments be defined as a binary cross-entropy training loss, in accordance with equation (2):

$$L2 = -\frac{1}{n}\Sigma_i(y_i \log(\hat{y}_i) + (1 - y_i)\log(1 - \hat{y}_i)) \qquad (2)$$

where $y_i$ is the ground truth label of the known group that instance $i$ belongs to and $\hat{y}_i$, is the predicted label, for example the outputs of the classification layer. This step modifies the embedding H 204 from the unsupervised training step to include information about known groups, which may improve the separability of the embedding and facilitate the discovery of unknown groups, as will be discussed later on with reference to Figure 4. As labels are used for the classification task, the training at step 304 is supervised.

In some embodiments, step 104 is a three-stage process, as shown in Figure 3C. In such embodiments, steps 302 and 304 are followed by a step 306, in which the autoencoder network 200 is trained using clustering loss L3. Step 306 further optimises the embedding H 204 for clustering. Clustering loss L3 may in some embodiments be defined in accordance with equation (3):

$$L3 = \Sigma_i\Sigma_j \, p_{ij} \log \frac{p_{ij}}{q_{ij}} \qquad (3)$$

where $j \in \{1, 2 \dots k\}$ represents the index of a cluster from a total of $k$ clusters. $q_{ij}$ represents the probability of assigning instance $i$ of the input data to cluster $j$ (soft assignment) and $p_{ij}$ represents an auxiliary target distribution used to refine the clusters by learning from the high confidence assignments. At step 306, the autoencoder network 200 is optimised at the same time as the clustering through an iterative algorithm that alternates between two steps. In the first step, the soft assignment $q_{ij}$ between the embedding H 204 and the clusters is computed. In the second step, the embedding H 204 is optimised at the same time as the cluster centres by learning from the high confidence assignments with the help of the auxiliary target distribution $p_{ij}$ using clustering loss L3.

To facilitate an improved clustering, the auxiliary target distribution $p_{ij}$ is chosen to have the following two properties: put more emphasis on high confidence assignments and normalise the contribution of each cluster centre to the clustering loss in order to prevent large clusters from distorting the embedded space. Optionally, the target distribution may be chosen to strengthen predictions (measured through a clustering quality criteria such as cluster purity).

By way of non-limiting example, the auxiliary target distribution $p_{ij}$ may be computed by squaring $q_{ij}$ and normalising by frequency per cluster in accordance with equation (4):

$$p_{ij} = \frac{q_{ij}^2/f_j}{\Sigma_{j'} q_{ij'}^2/f_{j'}}, \qquad (4)$$

where $f_j = \Sigma_i q_{ij}$ is the soft cluster frequency of cluster $j$. By way of non-limiting example, the soft assignment distribution $q_{ij}$ may be computed using the Student t-distribution to measure the similarity between an embedding $h_i$ of an instance $X_i$ of the input data and a centre $\mu_i$ of cluster j, in accordance with equation (5):

$$q_{ij} = \frac{(1 + \|h_i - \mu_j\|^2/\alpha)^{-\frac{\alpha+1}{2}}}{\Sigma_{j'}(1 + \|h_i - \mu_{j'}\|^2/\alpha)^{-\frac{\alpha+1}{2}}}, \qquad (5)$$

where a represents the degrees of freedom of the Student's t-distribution. The Student t-distribution is convenient because it is non-parametric, but it could be appreciated that other parametric distribution or other similarity kernels may be used. Equally, different target distributions $p_{ij}$ may be used.

The network is trained at step 306 with loss L3 to match the soft assignments $q_{ij}$ to the target distribution $p_{ij}$. By learning from its own high-confidence predictions, the training at step 306 can be seen as a form of self-training. Step 104 improves the separability of clusters through self-training. This training step corresponds to the clustering step of DEC and in some embodiments may be implemented in the same way, so that these embodiments can be seen as a modification of DEC in which a supervised classification training step is interspersed between the training steps of DEC.

In some embodiments, steps 104 and 106 of process 100 may be combined into a single step. In these embodiments, the clusters can be defined using the soft assignments $q_{ij}$ from clustering loss L3 and therefore, in these embodiments, the clustering of input data is a by-product of step 104.

In some embodiments, step 104 of processing the input data to learn the embedding H 204 is done by performing steps 302 and 304 simultaneously. This can be achieved by combining reconstruction loss L1 and classification loss L2 as a single loss function L in accordance with equation (6):

$$L = w_1 L1 + w_2 L2 \qquad (6)$$

where $w_1$ and $w_2$ represent the weights assigned to each loss and can be set according to what the optimal embedding would be depending on each problem of interest. Alternatively, steps 302 and 304 can be performed simultaneously by applying L1 and L2 sequentially for one epoch of training each, for example alternatingly or on a more complex schedule, for example with different numbers of repetitions for each respective update during an epoch (or, put differently, alternating epochs each with a respective one of the cost function or different proportions of respective epochs intermingled).

In some embodiments, step 104 of processing the input data to learn the embedding H 204 is done by performing steps 302, 304 and 306 simultaneously. This can be achieved by combining reconstruction loss L1, classification loss L2 and clustering loss L3 as a single loss function L in accordance with equation (7):

$$L = w_1 L1 + w_2 L2 + w_3 L3 \qquad (7)$$

where $w_1$, $w_2$ and $w_3$ represent the weights assigned to each loss and can be set according to what the optimal embedding would be depending on each problem of interest. Alternatively, steps 302, 304 and 306 can be performed simultaneously by applying L1, L2 and L3 sequentially for one epoch of training each, for example alternatingly or on a more complex schedule, for example with different numbers of repetitions for each respective update during an epoch (or, put differently, alternating epochs each with a respective one of the cost function or different proportions of respective epochs intermingled).

In some embodiments, the order in which steps 304 and 306 in Figure 3C are executed may be swapped.

By combining the unsupervised reconstruction and, in some embodiments clustering, with the supervised classification task, process 100 produces a cluster optimised embedding of high-dimensional input data that is adapted to provide separation of clusters in the embedded space. Figure 4 depicts a schematic visualisation of the benefits of semi-supervised clustering process 100 over other clustering methods. The left plot illustrates an embedding 402 based on the DEC method. The DEC embedding makes it difficult to distinguish between different clusters, even between those of known groups 1 and 2, although there is some separation between the groups. The right plot illustrates an embedding 404 according to a disclosed embodiment. The embedding 402 allows for a clearer separation of the embedded space. It can be seen that the additional supervised training results in an improved separation in the embedding of the known groups. However, surprisingly, adapting the embedding to separate the known groups also facilitates the adaptation of the embedding to discover unknown subgroups during clustering updates However, it can be noted that advantages of better separation in the embedding may arise solely due to the classification update, whether the embedding is further adapted using a clustering update or not.

*Detailed Example: Stratification of heart failure patients*

In the detailed example described below, the semi-supervised clustering of process 100 was performed on EHR data from heart failure patients. At step 102, the input data was preprocessed to first exclude patients with more than 60% of record data missing and then to fill in the missing values for the remaining patients. Missing values were filled in using the BRITS method, illustrated in study W. Cao, H. Zhou, D. Wang, Y. Li, J. Li, and L. Li, "BRITS: Bidirectional recurrent imputation for time series,"Adv. Neural Inf. Process. Syst., no. NeurIPS, pp. 6775-6785, 2018, incorporated by reference in this disclosure. At step 104, the embedding was obtained by applying steps 302, 304 and 306 sequentially.

The autoencoder network architecture used was the one illustrated in Figure 2 with layers of 13, 1000, 500, 3, 500, 1000 and 13 neurons. The autoencoder was trained for 50 epochs at step 302, for 10 epochs at step 304 and for 200 epochs at step 306. After creating a clustering-optimised embedding using the iterative two-step algorithm in step 306, the resulting embedding is clustered into unknown subgroups in step 106 using agglomerative clustering, a type of hierarchical clustering. See Rokach, Lior, and Oded Maimon. "Clustering methods." Data mining and knowledge discovery handbook. Springer, Boston, MA, 2005. 321-352, incorporated by reference in this disclosure. For comparisons purposes, DEC was also applied to the same dataset. To assess whether the discovered subgroups improve the patient representation, the log odds ratio is computed for each subgroup. The log odds ratio is a commonly used statistic that quantifies the strength of the association between events. Survival rates over time were determined for the respective subgroups of each cluster. Specifically, Kaplan-Meier curves were calculated for each cluster subgroup to assess if the clusters were an indicator of patient survival. See Kaplan, Edward L., and Paul Meier. "Nonparametric estimation from incomplete observations." Journal of the American statistical association 53.282 (1958): 457-481, incorporated by reference in this disclosure.

*Dataset for Detailed Example*

An EHR dataset of 2,291 patients with a diagnosis of heart failure and 2,196 patients with any other diagnosis (representing the control group) was analysed. The EHR of each patient is composed of physiological data from one or more hospital admissions associated with a diagnosis code. Each admission data contains 13 features representing laboratory results and vital sign measurements: systolic blood pressure, diastolic blood pressure, heart rate, oxygen saturation, temperature, alanine aminotransferase, creatinine, c-reactive protein, platelets, potassium, sodium, urea and white blood cells. A number of these metrics have been linked to heart failure prognosis and were therefore used for the supervised classification task of heart failure versus control. It will be appreciated that, while the specific input data structure with the mentioned 13 features is introduced in the context of a detailed example experiment, this data structure can be used in conjunction with any of the aspects and embodiments described above.

*Results for Detailed Example*

According to the embodiment illustrated in Figure 3C, a semi-supervised clustering model was trained on the EHR dataset described above to cluster patients in the embedded space while also learning to discriminate between known groups (heart failure versus control) in order to improve the characterisation of unknown subgroups.

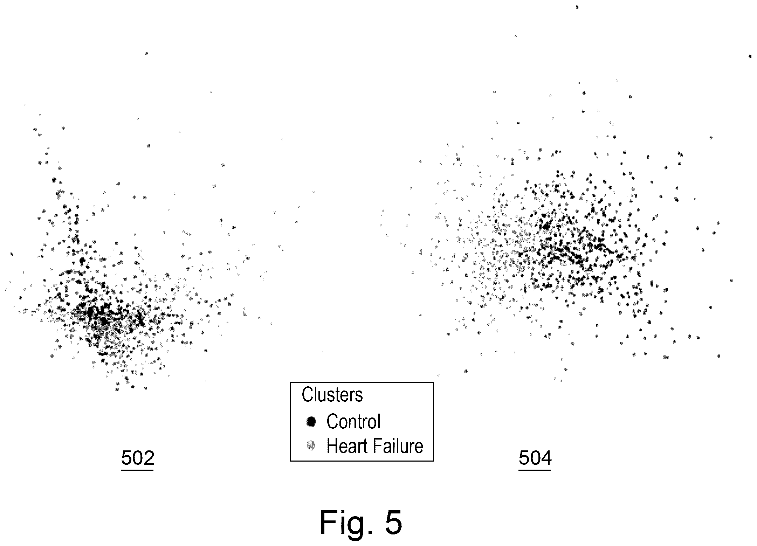

Figure 5 shows the separability of the heart failure and control groups using the DEC embedding 502 and the semi-supervised clustering embedding 504 obtained according to the described embodiment for the detailed example. Figure 5 demonstrates visually that the separability of the two patient groups is improved when using the semi-supervised clustering.

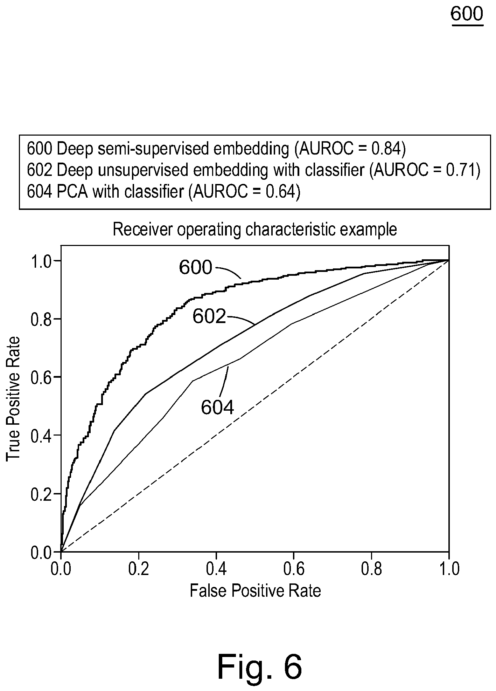

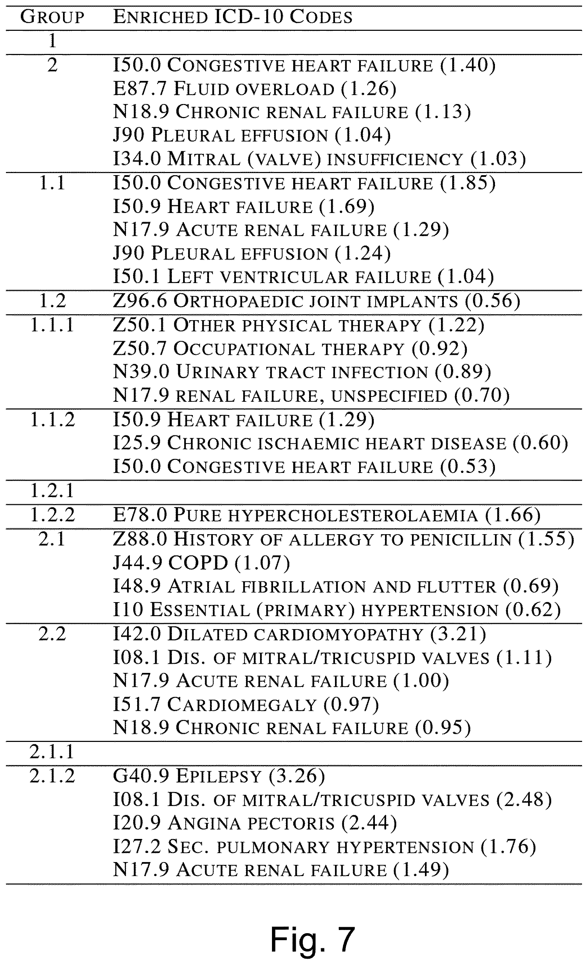

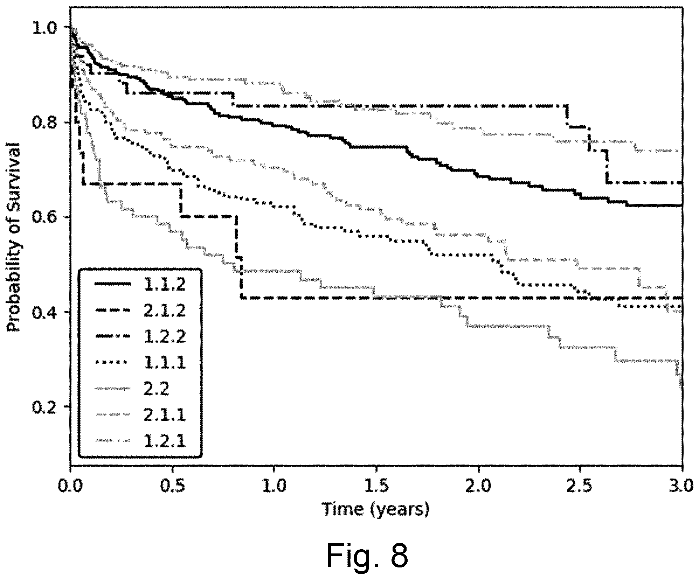

Figures 6 and 7 quantitatively show that the described semi-supervised clustering approach can achieve good discrimination between known heart failure and control groups whilst simultaneously finding unknown patient subgroups. Figure 6 shows the Area Under the Receiver Operating Curve (AUROC) results of discriminating between the two known patient groups using three low-dimensionality embeddings: a semi-supervised embedding 600 according to the embodiment described above (which performs classification at step 304 as part of training), a DEC unsupervised embedding with a random forest classifier 602 and a PCA embedding with a random forest classifier 604. The results show that the semi-supervised embedding 600 can lead to a better group discrimination than embeddings learnt using known unsupervised embedding methods 602 and 604. Figure 7 shows that the semi-supervised clustering technique can enable the discovery of unknown clusters representing statistically significant diagnosis which frequently occur in one group compared to a pairwise comparison to another group. In Figure 7, the first split is between the heart failure group (group 2) and the non-heart failure control group (group 1). Then, the two groups are split into subgroups. For example, the heart failure group 2 is further split into subgroup 2.1, which includes patients with hypertension, COPD, allergy to penicillin and atrial fibrillation, and subgroup 2.2, which includes patients with dilated cardiomyopathy, renal failure and other three symptoms. Next, the subgroups are further split. For example, subgroup 2.1 is further split into subgroups 2.1.1 and 2.1.2, each subgroup being associated with certain diagnosis codes which occur within the respective subgroup. For example, subgroup 2.1.2 includes a statistically significant diagnosis of epilepsy (code G40.9). The log odds ratio for each diagnosis is shown between the brackets. If the probability of a diagnosis occurring in one group is greater than the probability of occurring in another group, then the log odds ratio is greater than zero. Therefore, with respect to the epilepsy diagnosis G40.9 in subgroup 2.1.2, the log odds ratio equal to 3.26 shows that diagnosis G40.9 is more likely to occur in subgroup 2.1.2 than in subgroup 2.1.1. These results confirm that the semi-supervised clustering process can facilitate the discovery of clinically relevant subgroups from EHR data. Figure 8 shows the survival rates over time for the subgroup of the cluster in Figure 7. The separation between survival curves of the discovered subgroups indicates the potential clinical utility of the semi-supervised clustering process.

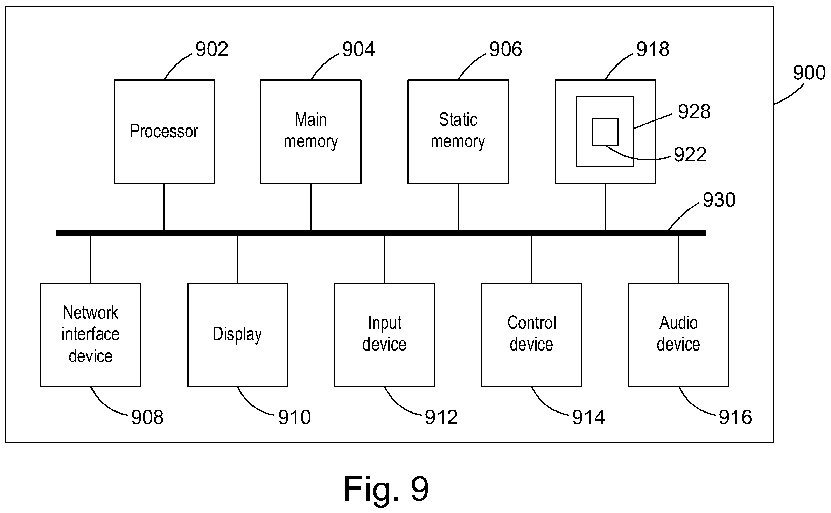

Figure 9 illustrates a block diagram of one implementation of a computing device 900 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 900 includes a processing device 902, a main memory 904 (e.g., read-only memory (ROM), flash memory, dynamic random access memory (DRAM) such as synchronous DRAM (SDRAM) or Rambus DRAM (RDRAM), etc.), a static memory 906 (e.g., flash memory, static random access memory (SRAM), etc.), and a secondary memory (e.g., a data storage device 918), which communicate with each other via a bus 930.

Processing device 902 represents one or more general-purpose processors such as a microprocessor, central processing unit, or the like. More particularly, the processing device 902 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 902 may also be one or more special-purpose processing devices such as an application specific integrated circuit (ASIC), a field programmable gate array (FPGA), a digital signal processor (DSP), network processor, or the like. Processing device 902 is configured to execute the processing logic (instructions 922) for performing the operations and steps discussed herein.

The computing device 900 may further include a network interface device 908. The computing device 900 also may include a video display unit 910 (e.g., a liquid crystal display (LCD) or a cathode ray tube (CRT)), an alphanumeric input device 912 (e.g., a keyboard or touchscreen), a cursor control device 914 (e.g., a mouse or touchscreen), and an audio device 916 (e.g., a speaker).

The data storage device 918 may include one or more machine-readable storage media (or more specifically one or more non-transitory computer-readable storage media) 928 on which is stored one or more sets of instructions 922 embodying any one or more of the methodologies or functions described herein. The instructions 922 may also reside, completely or at least partially, within the main memory 904 and/or within the processing device 902 during execution thereof by the computer system 900, the main memory 904 and the processing device 902 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining," "identifying", "providing", "applying", "training" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A computer-implemented method of training an artificial neural network, the method comprising:

   providing an artificial neural network comprising a feedforward autoencoder and a classification layer, the feedforward autoencoder comprising an input layer of neurons, an output layer of neurons, an embedding layer of neurons between the input and output layers, one or more non-linear intermediate layers of neurons between the input and embedding layer, one or more further non-linear intermediate layers of neurons between the embedding and output layer and respective network weights for each layer, wherein the number of neurons in the embedding layer is less than the number of neurons in the input layer;

   providing data units within a data space to have a data value for each dimension of the data space and a classification label associated with each data unit indicating one of a plurality of groups to which each data sample belongs;

   applying the data values of each data unit to corresponding neurons of the input layer to produce an output of the neurons in the output layer, wherein neuron outputs of the neurons in the embedding layer resulting from the application of each data unit provide an embedding of the data unit and wherein the embedding has fewer dimensions than the data space;

   using the embedding of each data unit to provide an input to the classification layer to produce a classification for each data unit as an output of the classification layer;

   comparing the classification and classification label to determine a classification loss for each data unit;

   comparing the data values and the output of the respective neurons in the output layer to determine a reconstruction loss for each data unit; and

   training the artificial neural network to reduce the reconstruction loss and the classification loss across the data units.

2. The method of claim 1 comprising using the respective embeddings of the data units as an input to a clustering algorithm to assign each data unit to a respective one of a plurality of clusters by optimising a clustering cost function, wherein training the artificial neural network further comprises using a value of the clustering cost function for each data unit to train the autoencoder.

3. The method of claim 2 comprising using the value of the clustering cost function to train the auto encoder subsequent to training the artificial neural network to reduce the reconstruction loss and the classification loss across the data units.

4. The method of any preceding claim, comprising training the artificial neural network to reduce the classification loss subsequent to training the autoencoder to reduce the reconstruction loss.

5. The method of claim 4, wherein training the artificial neural network comprises updating all layers of the autoencoder to reduce the reconstruction loss and fixing at least one of the intermediate layers while updating the remaining layers of the artificial neural network to reduce the classification loss subsequent to training the autoencoder to reduce the reconstruction loss.

6. The method of any preceding claim, wherein each data unit is representative of phenotypic data of a respective patient from a population of patients, wherein the population comprises at least two groups and the data label identifies the group the patient belongs to.

7. The method of claim 6, wherein the phenotypic data is obtained from an electronic health record.

8. The method of claim 6 or claim 7, wherein the phenotypic data is physiological data.

9. The method of claim 8, wherein the physiological data comprises three or more of: systolic blood pressure, diastolic blood pressure, heart rate, oxygen saturation, temperature, alanine aminotransferase, creatinine, c-reactive protein, platelets, potassium, sodium, urea and white blood cells.

10. The method of any one of claims 6 to 9, wherein the at least two groups comprise a pathology group of patients with an identified pathology and a control group of patients without the pathology.

11. The method of any preceding claim, wherein applying the data values of each data unit to corresponding neurons of the input layer comprises adding noise to the data values.

12. A computer-implemented method of classifying a data unit as belonging to a group of data units of a plurality of groups of data units, the method comprising providing a neural network trained according to any one of the preceding claims, applying the data unit to the input layer, obtaining the embedding for the data unit and using the embedding to classify the data unit.

13. A computer implemented method of identifying subgroups comprising training a neural network in accordance with the method of any one of claims 1 to 11, the method comprising clustering the embeddings of the data units into a plurality of clusters and identifying a subgroup of at least one of the groups as corresponding to one of the clusters.

14. The method of claim 13 when dependent on claim 10, wherein the at least one of the groups is the pathology group.

15. One or more computer-readable media encoding computer instruction that, when executed on a computing device, implement a method as claimed in any one of the preceding claims.

100

receive and preprocess
input data
102

↓

process input data to
learn an embedding
104

↓

cluster input data in the
embedded space
106

## Fig. 1

200

Encoder
202

Decoder
206

Input
X

Embedding
H
204

Output
X̂

13    1000    500    3    500    1000    13

No. of neurons

## Fig. 2

104

train network using
reconstruction loss L1
302

↓

train network using
classification loss L2
304

## Fig. 3A

Fig. 3B

104

train network using
reconstruction loss L1          302

train network using
classification loss L2           304

train network using
clustering loss L3               306

# Fig. 3C

Unknown
Group 1a

Unknown
Group 1b

Known
Group 1

Unknown
Group 2a

Unknown
Group 1b

Known
Group 2

402

404

# Fig. 4

Clusters
● Control
◉ Heart Failure

502     504

Fig. 5

600

600 Deep semi-supervised embedding (AUROC = 0.84)
602 Deep unsupervised embedding with classifier (AUROC = 0.71)
604 PCA with classifier (AUROC = 0.64)

Receiver operating characteristic example

Fig. 6

| GROUP | ENRICHED ICD-10 CODES |
|-------|------------------------|
| 1 | |
| 2 | I50.0 CONGESTIVE HEART FAILURE (1.40) <br> E87.7 FLUID OVERLOAD (1.26) <br> N18.9 CHRONIC RENAL FAILURE (1.13) <br> J90 PLEURAL EFFUSION (1.04) <br> I34.0 MITRAL (VALVE) INSUFFICIENCY (1.03) |
| 1.1 | I50.0 CONGESTIVE HEART FAILURE (1.85) <br> I50.9 HEART FAILURE (1.69) <br> N17.9 ACUTE RENAL FAILURE (1.29) <br> J90 PLEURAL EFFUSION (1.24) <br> I50.1 LEFT VENTRICULAR FAILURE (1.04) |
| 1.2 | Z96.6 ORTHOPAEDIC JOINT IMPLANTS (0.56) |
| 1.1.1 | Z50.1 OTHER PHYSICAL THERAPY (1.22) <br> Z50.7 OCCUPATIONAL THERAPY (0.92) <br> N39.0 URINARY TRACT INFECTION (0.89) <br> N17.9 RENAL FAILURE, UNSPECIFIED (0.70) |
| 1.1.2 | I50.9 HEART FAILURE (1.29) <br> I25.9 CHRONIC ISCHAEMIC HEART DISEASE (0.60) <br> I50.0 CONGESTIVE HEART FAILURE (0.53) |
| 1.2.1 | |
| 1.2.2 | E78.0 PURE HYPERCHOLESTEROLAEMIA (1.66) |
| 2.1 | Z88.0 HISTORY OF ALLERGY TO PENICILLIN (1.55) <br> J44.9 COPD (1.07) <br> I48.9 ATRIAL FIBRILLATION AND FLUTTER (0.69) <br> I10 ESSENTIAL (PRIMARY) HYPERTENSION (0.62) |
| 2.2 | I42.0 DILATED CARDIOMYOPATHY (3.21) <br> I08.1 DIS. OF MITRAL/TRICUSPID VALVES (1.11) <br> N17.9 ACUTE RENAL FAILURE (1.00) <br> I51.7 CARDIOMEGALY (0.97) <br> N18.9 CHRONIC RENAL FAILURE (0.95) |
| 2.1.1 | |
| 2.1.2 | G40.9 EPILEPSY (3.26) <br> I08.1 DIS. OF MITRAL/TRICUSPID VALVES (2.48) <br> I20.9 ANGINA PECTORIS (2.44) <br> I27.2 SEC. PULMONARY HYPERTENSION (1.76) <br> N17.9 ACUTE RENAL FAILURE (1.49) |

# Fig. 7

Fig. 8

EP 3 872 711 A1

Fig. 9

Europäisches
Patentamt

European
Patent Office

Office européen
des brevets

## EUROPEAN SEARCH REPORT

Application Number

EP 20 16 0197

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHOU WEN'AN ET AL: "Deep Embedded Clustering With Adversarial Distribution Adaptation", IEEE ACCESS, vol. 7, 28 August 2019 (2019-08-28), pages 113801-113809, XP011742441, DOI: 10.1109/ACCESS.2019.2935388 [retrieved on 2019-08-22] * the whole document * | 1-15 | INV. G06N3/04 G06N3/08 G06N20/00 |
| X | MIN ERXUE ET AL: "SU-IDS: A Semi-supervised and Unsupervised Framework for Network Intrusion Detection", 13 September 2018 (2018-09-13), ANNUAL INTERNATIONAL CONFERENCE ON THE THEORY AND APPLICATIONS OF CRYPTOGRAPHIC TECHNIQUES, EUROCRYPT 2018; [LECTURE NOTES IN COMPUTER SCIENCE; LECT.NOTES COMPUTER], SPRINGER, BERLIN, HEIDELBERG, PAGE(S) 322 - 334, XP047485226, ISBN: 978-3-642-17318-9 [retrieved on 2018-09-13] * the whole document * | 1-13,15 | |
| X | WEN XIE ET AL: "POLSAR Image Classification via Clustering-WAE Classification Model", IEEE ACCESS, vol. 6, 17 July 2018 (2018-07-17), pages 40041-40049, XP055725060, DOI: 10.1109/ACCESS.2018.2852768 * the whole document * | 1-15 | TECHNICAL FIELDS SEARCHED (IPC) G06N |

-/--

The present search report has been drawn up for all claims

| Place of search | Date of completion of the search | Examiner |
|---|---|---|
| The Hague | 27 August 2020 | Suarez Y Gonzalez, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 16 0197

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BEAULIEU-JONES BRETT K ET AL: "Semi-supervised learning of the electronic health record for phenotype stratification", JOURNAL OF BIOMEDICAL INFORMATICS, ACADEMIC PRESS, NEW YORK, NY, US, vol. 64, 12 October 2016 (2016-10-12), pages 168-178, XP029835872, ISSN: 1532-0464, DOI: 10.1016/J.JBI.2016.10.007 * the whole document * | 6-10,14 | |
| A | HASSANI KAVEH ET AL: "Unsupervised Multi-Task Feature Learning on Point Clouds", 2019 IEEE/CVF INTERNATIONAL CONFERENCE ON COMPUTER VISION (ICCV), IEEE, 27 October 2019 (2019-10-27), pages 8159-8170, XP033723464, DOI: 10.1109/ICCV.2019.00825 [retrieved on 2020-02-24] * the whole document * | 1-15 | |

TECHNICAL FIELDS
SEARCHED       (IPC)

The present search report has been drawn up for all claims

| Place of search | Date of completion of the search | Examiner |
|---|---|---|
| The Hague | 27 August 2020 | Suarez Y Gonzalez, R |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XIE, JUNYUAN ; ROSS GIRSHICK ; ALI FARHADI.** Unsupervised deep embedding for clustering analysis. *International conference on machine learning,* 2016
- **KINGMA, DIEDERIK P. ; MAX WELLING.** An introduction to variational autoencoders. *Foundations and Trends® in Machine Learning,* 2019, vol. 12.4, 307-392
- **VINCENT ; PASCAL et al.** Stacked denoising autoencoders: Learning useful representations in a deep network with a local denoising criterion. *Journal of machine learning research,* 11 December 2010, 3371-3408
- **HINTON, GEOFFREY E. ; RUSLAN R. SALAKHUTDINOV.** Reducing the dimensionality of data with neural networks. *science,* 2006, vol. 313.5786, 504-507
- **KINGMA, DIEDERIK P. ; JIMMY BA.** *Adam: A method for stochastic optimization,* 2014
- **XIE ; JUNYUAN ; ROSS GIRSHICK ; ALI FARHADI.** Unsupervised deep embedding for clustering analysis. *International conference on machine learning,* 2016
- **W. CAO ; H. ZHOU ; D. WANG ; Y. LI ; J. LI ; L. LI.** BRITS: Bidirectional recurrent imputation for time series. *Adv. Neural Inf. Process. Syst., no. NeurIPS,* 2018, 6775-6785
- Clustering methods. **ROKACH ; LIOR ; ODED MAIMON.** Data mining and knowledge discovery handbook. Springer, 2005, 321-352
- **KAPLAN ; EDWARD L. ; PAUL MEIER.** Nonparametric estimation from incomplete observations. *Journal of the American statistical association,* 1958, vol. 53.282, 457-481